# EUROPEAN PATENT APPLICATION

(11) **EP 4 074 296 A1**
(43) Date of publication of application: **19.10.2022**
(21) Application number: 21168745.4
(22) Date of filing: 16.04.2021
(51) Int. Cl.: A61F 13/495

(54) **HYGIENE ARTICLE COMPRISING SKIN PROTECTION SHEET AND ABSORBENT CORE WITH WELL POSITIONED OPENINGS**

(71) Applicant: Concepts For Success (C4S), 53881 Euskirchen (DE)
(72) Inventor: SCHMITZ, Christoph, 53881 Euskirchen (DE)
(74) Representative: Plischke, Manfred

(57) **Abstract**

The present invention relates to the field of hygiene appliances, in particular to hygiene articles comprising a means for reducing soiling of the skin of a wearer by body exudates such as faeces, urine, or menses. In particular, it relates to an article with a Skin Protection Sheet (SPS) that comprises a rear opening in registry with the anus during use, and a front opening in registry with a body exudate releasing body opening or genital organ. The openings are urged to the body by a pull means, whilst being spread open cross-directionally, allowing exudates to pass through. Such a hygiene article may suitably be a baby or adult diaper or pant, a training pant, or a menstrual pant.

## Description

### Field of the invention

The present invention relates to the field of hygiene appliances, in particular to hygiene articles comprising a means for reducing soiling of the skin of a wearer by body exudates such as faeces, urine, or menses by a skin protection sheet adapted to allow bodily exudates to pass through openings.

In particular, it relates to such an article exhibiting improved liquid handling capability by separating faeces from other bodily exudates.

Such a hygiene article may suitably be a baby or adult diaper or pant, a training pant, or a menstrual pant.

### Background

There are multiple attempts to address the problem of contaminating and/or irritating the skin of a wearer especially if a wearer is unable to control bowel movement or defecation.

In JP6538260 an absorbent article with hollow anal hump is described. The hump as may be made of sponge material is positioned in registry with the anus during use, supported thereby by an elastic member. The hollow hump comprises a pipe-like passage to guide faeces from the tip of the hump through the pipe-like passage of the hump and an opening in the liquid absorbent core to a faeces storage space. Whilst such a design may effectively separate faeces and urine, it may lead to discomfort during use. It also presents a challenge in the manufacturing and packaging of such an article.

In WO2020049109A1 (C4S, hereinafter referred to as "WO'109") there is disclosure of a hygiene article comprising a skin protection sheet that comprises a discontinuity as a faeces passageway, such as an opening or a slit. Whilst being a flat structure for manufacturing and packaging, upon donning, the sheet and the discontinuity are urged towards the anus by a pull means and spread open by a spreading means, such that faeces can pass through the discontinuity but are separated from the skin of the wearer elsewhere. However, it has been discovered that there is still a need for enhancing the performance of urine handling, as may be desired for product variants as may be specifically adapted for particular user needs, such as male or female user, or user that suffer to a differing degree from urine incontinence and/or faecal incontinence.

Such improvements should be executable by a process on a manufacturing equipment that is easy to design and operate, and which can be readily adapted to various executions of these specific design variants.

### Summary

The present invention is a hygiene article for being worn on the lower torso of a wearer, exhibiting a front and a rear waist region along a length / longitudinal / x - direction and separated by a crotch point region, comprising a crotch point adapted to be positioned in the perineal region of the wearer during used, and a width / cross-directional / y-direction, perpendicular thereto and corresponding to the left-right direction of the wearer, and a z- or thickness direction perpendicular to the x- and y-direction.

The article comprises
- a skin protection sheet (SPS) adapted to separate bodily exudates from the skin of a wearer,
   ∘ the SPS comprising
   ■ a rear opening adapted to be positioned in registry with the anus and
   ■ a front opening to be positioned in registry with a bodily exudate releasing body opening or a genital organ,
   ■ whereby the rear and front opening do not extend into each other and are positioned opposite to each other relative to the crotch point of the article,
- a backsheet adapted to retain the bodily exudates,
- an absorbent core adapted to absorb liquids of the bodily exudates and being positioned z-directionally between the SPS and the backsheet,
- a pull and spreading strip (PSS) adapted to
   ∘ urge the SPS into the anal cleft of a wearer during the article's intended use,
   ∘ and to maintain simultaneously the first and the second opening(s) of the SPS cross-directionally open during the article's intended use,

An absorbent core extends from said crotch point region longitudinally towards and under, optionally beyond at least one, preferably both of said front and rear opening of said SPS, and comprises at least one core opening in registry with at least one of the front and rear opening(s) of the SPS, and is connected to the SPS and directly or indirectly to the PSS in the proximity of the opening(s), preferably by melt-fusion bonding, more preferably ultrasonic bonding, such that opening of the absorbent core is maintained cross-directionally open upon the intended use, so as to allow exudates to pass through the SPS and the absorbent core.

Such a hygiene article may further exhibit a first manufacturing configuration wherein essentially all elements are essentially planar, and a second in-use configuration adopting a body conforming U-shape, by the longitudinally extending center line extending from the front or rear waist region of a wearer through the crotch point region into the opposite waist region of the wearer.

The openings may be formed by separation lines extending longitudinally along a longitudinal centerline of the article in the manufacturing configuration, or by removing materials from the absorbent core, SPS, and PSS.

Preferably, the absorbent core is essentially free of particulate material in the cross-directional proximity of the openings. Further, the absorbent core may comprise one or more seal lines in the proximity of the separation lines.

Optionally, the absorbent core comprises a first surface adapted for handling urine and an opposite surface adapted for handling faeces. The article may exhibit a capacity of more than about 50 ml, or more than about 100 ml, or more than about 200 ml, or more than about 300 ml or more than about 400 ml, or more than about 500 ml, or less than about 2000 ml, determined according to the Centrifuge Capacity Test.

The SPS may be selected from the group consisting of hydrophilized nonwoven material, hydrophobic nonwovens, aperture nonwovens, films, apertured films, and combinations thereof.

In a first execution, the core opening may be in registry with the front SPS opening, the core may be attached to the SPS in the proximity of the front SPS opening, and the core may be essentially unattached to the SPS in the proximity of the rear SPS opening.

In a second execution, the core opening may be in registry with the rear SPS opening, the core may be attached to the SPS in the proximity of the rear SPS opening, and the core may be essentially unattached to the SPS in the proximity of the front SPS opening.

In yet a further execution, the core may comprise a front and a rear opening in registry with the respective SPS openings, the core may be attached to the SPS in the proximity of the front and rear opening, and the article further comprises an exudate separation sheet (ESS) connected to the core in the crotch region longitudinally between the front and rear openings, which are positioned between the absorbent core and the backsheet, and extending longitudinally forward or rearward, so as to extend over one of the front and rear openings, but not the other.

### Brief description of the Figures

Fig. 1 depicts the lower torso of a wearer to which an article according to the present invention may be donned.
Fig. 2A to C depict an execution of articles according to the present invention based on the prior art WO' 109 and its fit on the lower torso of a wearer.
Fig. 3A to C absorbent articles according to prior art.
Fig. 4 A to E depict absorbent articles according to the present invention.

Same numerals in various figures refer to the same elements or features. Figures are schematically only and not scaled.

### Detailed description

The present invention relates to a hygiene absorbent article comprising a skin protection sheet, hereinafter abbreviated as "SPS". Such an article of manufacture is typically applied to the lower torso of a human, aiming at reducing soiling of the skin by body exudates, in particular for capturing faeces as well as other exudates such as during menstruation or when a wearer is not able to control urination.

Such hygiene articles further comprise a backsheet that retains the body exudates and prevents soiling of the environment, such as clothing or bedding, and an absorbent core for immobilizing liquid exudates. An article according to the present invention is particularly adapted to separate faeces and other exudates from each other, by guiding these exudates to respective opposite portions of the absorbent core such as when contact of urine and faeces should be prevented or core portions adapted to receive urine should not be contaminated by faeces. Thus, suitable hygiene articles may be diapers for babies or adults, be these of the "open type" with closure means such as tapes, or be these of the "pants type" closed at the sides so as to create a belt like system around the waist, training pants or fixation pants made of film or other materials. Suitable hygiene articles include diapers in an "all-in-one" execution, i.e. with combined liquid absorbency and liquid impermeable cover, or "two-piece", i.e., separate means for addressing liquid absorbency and liquid barrier function, or "inserts", wherein an absorbent article may be equipped with a replaceable absorbent pad. Other two-piece executions include the combination of an absorbent pad with fixation mean, such as a stretchable cover, such as a net pant. An article according to the present invention may also be used in combination with a conventional absorbent diaper or pant, e.g. to enhance bowel movement handling.

Hygiene articles may be disposable, i.e. discarded and/or further treated in an environmentally friendly and sound manner and may also be made from or comprise re-usable materials.

A hygiene article according to the present invention can be in a "manufacturing configuration" which refers to a state of being produced in a manufacturing system, preferably a high speed manufacturing system, allowing to produce more than hundred pieces per minute, more than 300 pieces per minute even more than 600 pieces per minute or even more than 1000 pieces per minute. At the end of the manufacturing line, the hygiene article may be brought into a "packaging configuration", such as by being cut and folded, in which it is delivered to a user, as may be the wearer or a caretaker, who will then bring the article into a "pre-use configuration" such as by unfolding it. Upon donning on a wearer, the article takes an "in-use configuration". The SPS may be combined with a hygiene article precursor at the manufacturing stage, or by a user e.g. when establishing the "pre-use configuration".

Typically, an absorbent article exhibits a width or cross-direction or y-direction, corresponding to a left-right orientation of a user during its intended use. Further, the length, longitudinal or x-direction of the article extends perpendicularly thereto, and in an in-use configuration from a first waist region, e.g. the rear waist region, through the crotch region to the opposite waist region, whereby the respective regions of the article correspond to the body regions of a wearer. Thus, in this in-use configuration the longitudinal center line of an article will take a U-shape configuration, whilst in a manufacturing configuration the longitudinal center line is often a straight line. Further, the article exhibits a thickness or z-direction, perpendicular to the x-, and y- directions. The overall length of the article in an in-use configuration corresponds to the outermost line of the article, which is the backsheet as described below, following this U-shape, thus stretching from the rear waist margin of the article through the crotch region to the front waist margin. In a manufacturing configuration, where individual articles are not yet separated but represent an essentially endless sequence of connected article precursors, the overall length of an article is considered to correspond to the distance of marked-up or imaginary lines where the articles are separated from adjacent ones towards the end of the manufacturing process.

An article according to the present invention comprises an absorbent core, the design of which is not particularly limited, and as such well known to a person skilled in the art. Such a core may be made of a homogeneous material or material mix, and may exhibit two- or three-dimensional shape adapted to the intended use. Preferably, a suitable absorbent core comprises a first surface, which in conventional articles is typically oriented towards the wearer, that is optimized for receiving the bodily exudates, as may be achieved by materials for allowing fast acquisition and good distribution of the bodily exudates, often referred to as "acquisition / distribution layer", or ADL. The absorbent core further comprises a liquid storage portion or element, typically adjacent to the ADL for storing the bodily exudates. Such a storage portion comprises porous material, often fibrous materials, and preferably superabsorbent polymers, also referred to as SAP, often in particulate form. The ADL and the storage portion may be distinct material layers, optionally comprising sub-layers, but also an essentially unitary structure with regions with differing properties can be used. Optionally, the absorbent core may further comprise a wrapping material. In a preferred execution, the absorbent core exhibits a layered structure that exhibits sufficient CD-strength to withstand the treatment as described herein below.

A suitable absorbent core exhibits an absorbent capacity adapted to the intended use of the article, as may be determined according to the test methods described herein below. When tested according to the Free Swell Absorption capacity test, an article according to the present invention exhibits a capacity of at least 100 ml, or more than 200 ml, or more than 500 ml, or more than 1000 ml, typically less than about 5000 ml. Similarly, following the Centrifuge Capacity Test, the article exhibits a capacity of more than about 50 ml, or more than about 100 ml, or more than about 200 ml, or more than about 300 ml or more than about 400 ml, or more than about 500 ml, typically, however less than about 2000 ml.

The absorbent capacity of the article may be provided essentially through the absorbent core, which may also be evaluated according to appropriately adapted test methods, though additional elements may be included in the article, such as will be described herein below in more detail in the context of an absorbent Exudate Separation Sheet (ESS).

The capacity of absorbent materials may also be expressed as a basis capacity, which refers to the absorbent capacity, as determined for the absorbent material in analogy to the Free Swell or Centrifuge Capacity determination for the absorbent article, however based on the area of the absorbent material. Thus an absorbent material suitable for the present invention may exhibit a basis capacity of more than about 300 ml/m², or more than about 600 ml/m², or more than 1000 ml/m², or more than about 3000 1/m², or more than 5000 ml/m², or even more than 10,000 ml/m², but typically less than about 20,000 ml/m², as then the thickness increase during use may become uncomfortable.

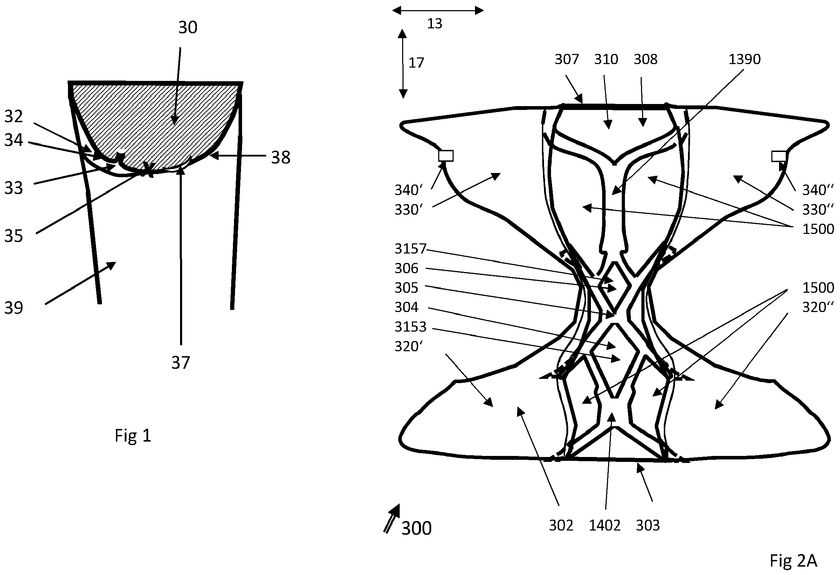

The principles of the present invention are now explained by referring to the figures, which should not be seen to be limiting, in particular not with regard to combining various features as described as exemplary executions. Fig. 1 depicts schematically a portion of the sagittal plane of a body, with lower torso 30, upper thigh of a leg 39 and buttocks 32, the latter being separated by the anal cleft 34. Further indicated are the anus 33 and the genital organs, here shown as labium 37, which is also the location of the male genital organs, scrotum and penis. Left and right groin clefts 38 extend from the crotch region forwardly. The perineum with a crotch point 35 is the region between anus and genital organs. The length of the perineal region has a person to person variability, and is also somewhat dependent on the gender and age. Typically, a range of 2.5 cm and 7.0 cm covers most of the adults, with a medium length of between 4.0 cm and 5.0 cm, both ranges being applicable to female and male persons. For babies, the perineal length is typically shorter, though not below about 1.0 cm. Thus, typically, the crotch point on a wearer is positioned between about 0.5 cm and about 4.0 cm forwardly of the forward end of the anus.

Within the present invention, the crotch point of an article 305 corresponding to the crotch point of a wearer 35 can be determined by placing an article on a wearer of the physical size for which the article is designed and who is in a fully upright standing position with his or her feet a shoulder width apart and then placing an extensible filament around the upper thighs in a figure eight configuration.

The point in the article corresponding to the point of intersection of the filament is considered to be the crotch point of the article. Further, the crotch point region of an article is longitudinally extending forward and rearward of the crotch point corresponding to the perineal region of a wearer. In absence of concrete figures for a particular user, the crotch point region is considered to extend at least 1 cm forward and rearward of the crotch point.

In order to explain the general functioning of the present invention, express reference is made to Fig. 2A to 2C, as known from the above referenced WO'109. Fig. 2A depicts schematically and nonlimiting a top view of an execution of article 300 according to the present invention in a pre-use configuration, with longitudinal direction 17 and width direction 13. The article 300 comprises a front region 302 with front cross-directionally extending margin 303 and a rear region 308 with rear cross-directionally extending margin 307 and a crotch region 305 longitudinally there between.

Further, front side panels 320' and 320" and rear side panels 330' and 330" extend laterally outwardly from a center piece 310. The side panels may be separate materials as connected to the backsheet material, or they may be lateral extensions of the backsheet material.

When reference is made to features, that are essentially symmetric to a longitudinal center line, as may be a "left-right" symmetry for an article, here shown for the side panels, the respective "left" or "right" features are denoted with single and double quotation marks, respectively, whilst in a general discussion of the feature, no quotation mark is used. The side panels may comprise closure means 340' and 340" that allow fitting of the article around the waist of a wearer by connecting front and rear side panels, respectively. As most of the materials suitable for being used in the present invention are essentially flat or web materials, they generally exhibit two surfaces separated by the thickness.

Within the present context, a first surface of the materials is generally oriented towards the wearer and the second or opposite surface away from the wearer or outwardly, at least in the crotch region, and even if certain portions of the materials are overfolded or are positioned towards the legs of a wearer.

In this exemplary execution, the article 300 further comprises a rear or anal opening 306 and a front or genital opening 304, both being cross-directionally spread open by a combined pull and spreading strip (PSS) 1390. In the front and rear portions, the PSS forms a Y-shape, with the stem of the "Y" directed towards the openings 306 and 304, respectively, and the legs of the "Y" directing away from the opening and laterally outward. A Skin Protection Sheet (SPS) 1500 is shown in the region of the center piece 310 except for the openings. An absorbent core 3150 is positioned under the SPS, i.e. away from the wearer, here shown visibly through front and rear openings 304 and 306.

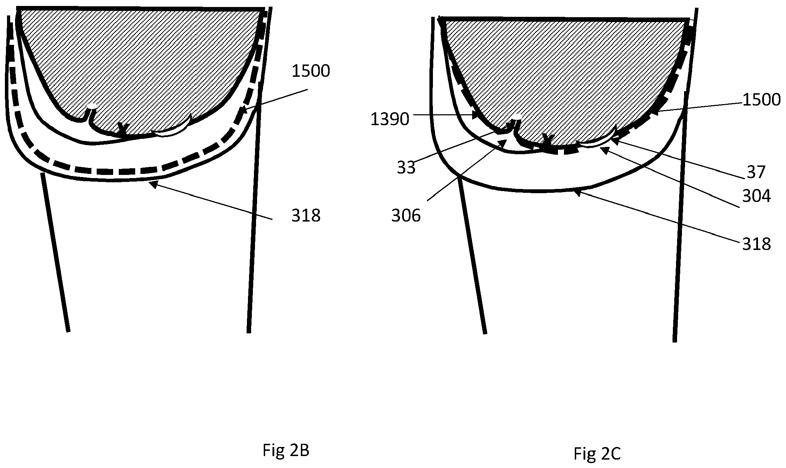

Fig. 2B depicts the portion of the sagittal plane of a body as in Fig. 1 with an article in a pre-use configuration as shown in Fig. 2A with the side panels not being fully outwardly folded and openings, PM, and SM omitted. The SPS 1500 and the backsheet 318 exhibit essentially the same longitudinal extension. Upon donning, and as depicted in Fig 2C, the outward folding of the sidepanels for closure of the article around the waist pulls the front and rear ends of the legs of the Y-shaped sections of the PSS 1390 laterally outward, thusly foreshortening the available longitudinal extension, and thusly urging the PSS 1390 as well as the SPS 1500 connected thereto into the anal cleft such that the openings are also urged towards the anus and genital organs whilst the backsheet remains spaced apart. At the same time, the spreading function of the PSS induces a lateral pull to the openings thereby widening their CD-extension at predetermined size, such that the rear opening fits tightly to the anus and the front opening is adapted to allow the genital organs to pass through. Thus exudates pass through the openings into the space created between the SPS and the backsheet, separated from the skin by the SPS.

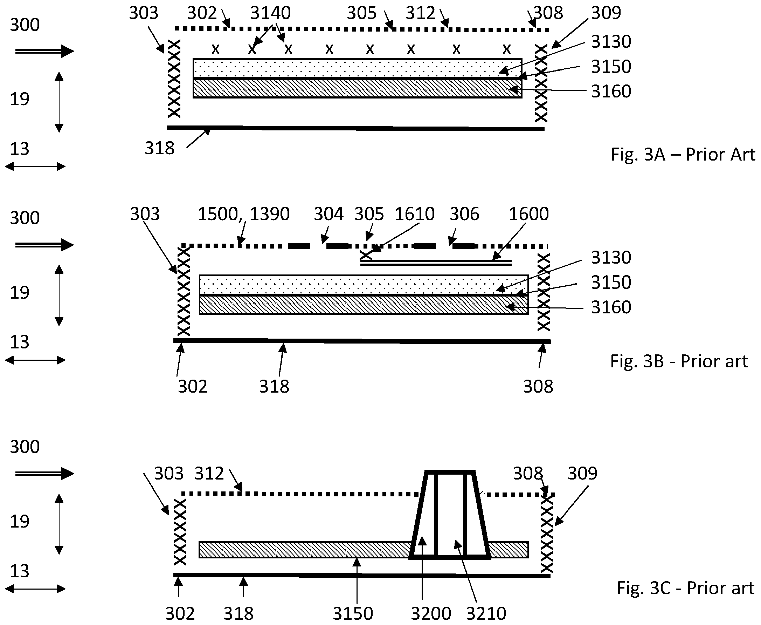

Having thus described the cooperative functionality of the SPS with the opening(s) and the pull and spreading strip, the following now focuses on the absorbent core underneath the SPS. Within the present context, it should be noted that the terms "underneath", "under", "below", "above" and similar refer to the positioning relative to a wearer during the intended use, such that the core is positioned further away from the wearer than the SPS, and a backsheet even further. Fig. 3A depicts schematically a x (13) -z (17) -directional view of a conventional diaper design 300, as currently common in the market. A topsheet 312 overlays an absorbent core 3150. Typically comprising a liquid storage element 3160 and a liquid handling layer, also referred to as acquisition - distribution layer (ADL) 3130. Typically, the topsheet 312 and the uppermost layer of the absorbent core are connected by adhesive connection 3120, as may be a spray or spiral glue, that should not unduly impede liquid penetration through the topsheet 312, that is typically executes with a non-permanent hydrophilization agent. A liquid impermeable backsheet 318 is connected in the front (302) and rear (308) portion of the articles by connecting lines 303 and 309, respectively, to the topsheet 312 The article typically comprises other elements, such as side extensions and / or cuffs, elastics, closure features, which are all well known in the art and not critical for the present invention. During use, urine and/ or menses are deposited onto the topsheet typically forwardly of the crotch point, whilst faeces is deposited in the rear portion behind the crotch point. However, as the faeces remains at least partially on the surface of the topsheet, urine and / or menses cannot reach the core through the faeces loaded surface portions. Needless to say that such designs do not at all address soiling of the skin by faeces.

An important improvement to reduce such soiling has been described in the above references WO'109, with selected features schematically depicted in Fig, 3B. The SPS 1500 combined with a combined pull and spreading strip 1390 is exemplarily shown with a front (304) and a rear (306) opening of the SPS such that both faeces and urine / menses can penetrate there through and are thusly kept away from the skin of the wearer. The execution as shown in Fig.3B further has an exudate separation sheet ESS 1600, being connected by ESS connection 1610 in the crotch region around crotch point 305 and extending rearwardly underneath the rear opening 306, thusly allowing to separate faeces and urine. However, at least upon loading of the article with faeces, the rear portion of the article cannot receive urine or menses via its upper surface, such that all liquid distribution has to be handled by the ADL 3130.

Yet a further approach known in the art, see above referenced JP6538260 is further explained by referring to Fig. 3C. An absorbent core 3150 comprises an opening 306 in the rear portion through which an anal hump 3200 protrudes, adapted to fit into the anal cleft for being in registry with the anal opening. A pipe-like passage 3210 may guide faeces through the core to the space between the core and the backsheet.

In contrast to these approaches, the present invention improves the liquid handling capability of a design known from WO'109 without inducing the negative effects of fit and comfort of the anal hump as known from JP6538260.

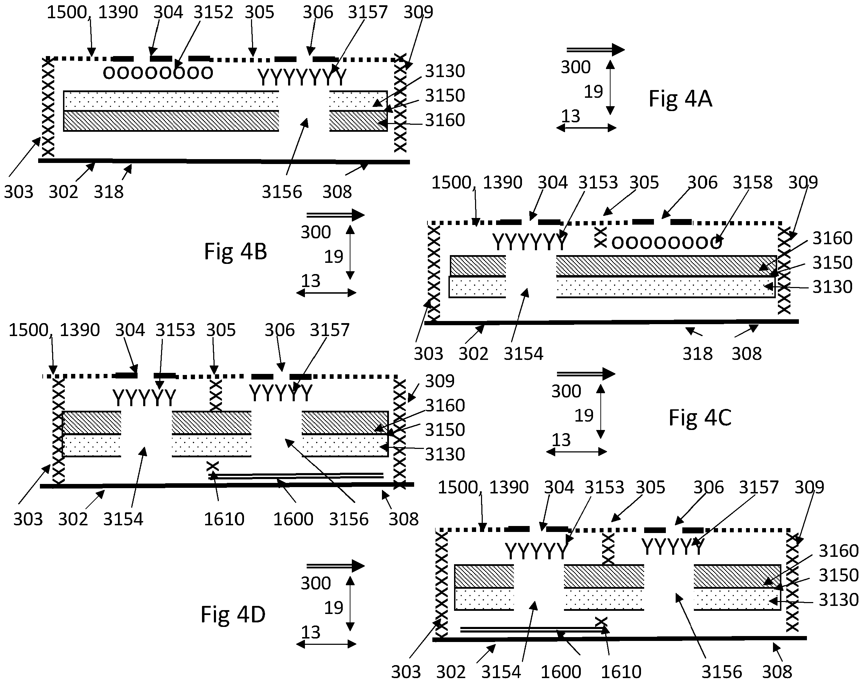

Referring to a first execution according to the present invention as depicted in Fig.4A, an absorbent article 300 comprises an absorbent core 3150, preferably including an ADL 3130 positioned towards the wearer, and a storage element 3160 positioned towards the backsheet. Combined SPS 1500 and PSS 1390 are connected in the front and rear region to the backsheet by connections 303 and 309, respectively, and have a front (304) and a rear (306) opening, spreading cross-directionally open upon donning as discussed in the above. In the region of the rear opening, the core is connected by a connection 3157 to the combined SPS 1500 and pull and spreading strip (PSS) 1390 just laterally outwardly of the opening. Further the core comprises a rear opening 3156 in registry with the rear opening 306 of the SPS. As for the SPS and the PSS, this opening may be made by applying a longitudinal separation line. Such a separation line may have an irregular or curved shape, however, it should have a predominant longitudinal extension, whereby, within the present context, the most longitudinally positioned points of the separation line are spaced apart more than the most laterally spaced apart points. Preferably, the separation line is a straight line, more preferably parallel to the longitudinal centreline of the article and in the proximity thereof, if not identical therewith. Upon donning, this separation line will spread open together with the spreading of the SPS opening in the proximity of the anus. Within the present context, the term "proximity" refers to distances of less than 15 mm or less than about 10 mm, or less than about 5 mm. Faeces can readily pass through, whilst urine or menses or the respective organs releasing urine or menses can penetrate through the front opening of the SPS 304 into a space 3152 without connections between core and SPS.

A second execution according to the present invention is depicted in Fig. 4B, wherein an absorbent article 300 comprises an absorbent core 3150, including an ADL 3130 positioned towards the backsheet, and a storage element 3160 positioned towards the wearer. Combined SPS 1500 and PSS 1390 are connected in the front and rear region to the backsheet by connections 303 and 309, respectively, and have a front (304) and a rear (306) opening, spreading cross-directionally open upon donning as discussed in the above. In the region of the front opening, the core is connected by a connection 3153 to the combined SPS and pull and spreading strip (PSS) just laterally outwardly of the opening. Further, the core comprises a front opening 3154 in registry with the front opening 304 of the SPS. As for the SPS and the PSS, this opening may be made by applying a longitudinal separation line. Upon donning, this separation line will spread open together with the spreading of the SPS opening in the proximity of the anus. Urine, menses, or urine or menses releasing organs can readily pass through to the backsheet side of the absorbent core 3150 and the ADL 3130 positioned there, such that its full surface is available for receiving liquid.

Yet a further execution according to the present invention is depicted in Fig. 4C and 4D, wherein an absorbent article 300 comprises an absorbent core 3150, preferably including an ADL 3130, positioned towards the backsheet and a storage element 3160 positioned towards the wearer. Combined SPS 1500 and PSS 1390 are connected in the front and rear region to the backsheet by connections 203 and 309, respectively, and have a front (304) and a rear (306) opening, spreading cross-directionally open upon donning as discussed in the above. In the region of the front and the rear SPS opening, the core is connected by a connection 3140 to the combined SPS and PSS just laterally outwardly of the openings. The core comprises a front (3154) and a rear (3156) opening in registry with the front and rear openings 304 and 306 of the SPS, respectively. As for the SPS and the PSS, these openings may be made by applying a longitudinal separation line. Upon donning, this separation lines will spread open together with the spreading of the SPS opening in the proximity of the anus. Further, the article comprises an exudate separation sheet 1600, as is positioned between the backsheet 318 and the absorbent core 3150 and connected to the core in the proximity of the crotch point 305 by connection 1610. The ESS may extend rearwardly, see Fig. 4C, and thus retains the faeces, as may pass through the rear opening 306 in the rear portion of the article, allowing the front part to be available for liquid reception. Alternatively, the ESS extends forwardly, thusly allowing faeces as passing through the rear opening 306 but keeping the front portion of the absorbent core free for liquid handling. These executions, however, are less preferred as they reduce the available surface for receiving liquid while being more complex in their making.

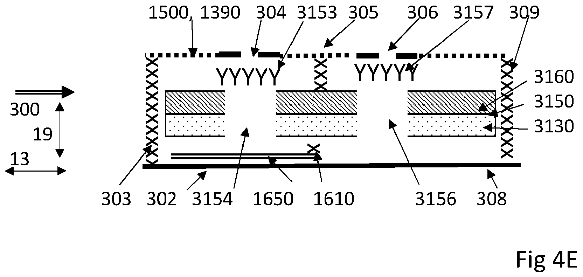

In yet a further execution as depicted in Fig. 4E, an absorbent ESS 1650 in a position as described in the context of Fig. 3D extending forwardly from the crotch point, may exhibit liquid absorbency, and may be constructed in analogy though not necessarily identical to an absorbent core and with materials as described in the above. The separation function may be achieved by liquid impermeability of the ESS, e.g. by including a liquid impermeable layer, and/or by providing sufficient liquid absorbent capacity therein, such as by exhibiting a basis capacity of more than about 100 ml/m², or more than about 200 ml/m². It should be noted that such an absorbent ESS increases the absorbent capacity of the hygiene article, but not of the absorbent core.

Urine, menses, or urine or menses releasing organs can readily pass through to the backsheet side of the absorbent core and the ADL positioned there, such that its full surface is available for receiving liquid.

For all executions, the absorbent cores 3150 most preferably comprise regions that exhibit different liquid handling functionality, as indicated in the above by separate ADL 3130 and liquid storage region 3150, the general executions thereof as well as multiple variations thereof being well known to a person skilled in the art of designing and manufacturing absorbent articles. The present invention does not imply particular restrictions to the design of such absorbent cores, except that preferably the absorbent core is free of particular material, such as superabsorbent particles, in the regions of the openings 3154, 3156 so as to avoid losses during manufacturing. Also, absorbent core 3150 or at least parts thereof are preferably enclosed by a wrapping material, such as nonwoven webs, so as to avoid particles to spill out and contact the skin of the wearer.

The skilled person will also realize that these regions do not need to be executed as co-extending layers, but may differ in length and/or width. Also, the thickness of the regions does not need to be x-y-directionally constant. The ADL and the liquid storage region may be made of pre-formed materials and be combined in the course of the process of manufacturing the articles, or may be made on line.

All discontinuities may also be executed as partly separated lines, where the separation is incomplete, such as by an intermittent cutting line or a perforation line (sometimes also referred to as "perf 'n pop"), such that the material remains connected during at least a part of the process, but is readily separated at least upon donning and in the in-use configuration. For the front and rear openings the x-y-extension will be formed from the respective separation lines upon the transition from the manufacturing configuration into the in-use configuration. Whilst the discontinuity may be and often preferably is executed as a separation line, such as a cut, it may also be formed by removing material from the web so as to form an opening already at this point in the manufacturing configuration. The connecting of the various elements may be achieved by any conventional means, such as without limitation heat or pressure bonding, or sonic, preferably ultrasonic bonding, though especially for the connections involving the overfolded regions, use of appropriate glues is preferred from a processability point of view.

As a skilled person will readily realize, the term "point", e.g. "connecting point", may exhibit a certain size, e.g. corresponding to the applied technology for connecting. Thus a glue-type connecting point includes a patch of glue sufficiently small to not impact functionality in the surrounding region.

Similarly, the term "line", e.g. "connecting line" may exhibit a certain width. Also the term encompasses an interrupted line, e.g. comprising a dotted glue or ultrasonic bonding line, as well as a bonding pattern, such as a line made of a multiplicity of (sub-) lines or a connecting point made of a multiplicity of smaller bond points. Similarly, the term "region" encompasses a long but narrow region that may also be seen as a "line".

Generally, the term "web" relates to any material which is essentially endless or continuous in one direction (generally denoted as "x-direction" or "machine direction"). Webs are often, but not necessarily, stored, supplied or used in roll form and thusly also sometimes denoted "roll goods".

Whilst these are then not "endless" in the strict sense of the word, their extension in this x-direction is significantly larger than in any other direction. By combining consecutive rolls or other batches, ("splicing") such webs can be considered "endless" for all practical purposes. Webs may be transported in a "batch" form, such as when a roll thereof is shipped, or they may follow a "web path", such as when the webs are unwound from a roll, as described hereinafter. Typical examples for webs are - without implying any limitation - plastic films or foils, optionally apertured, textiles, non-wovens, nets, or scrims.

The SPS and the pull and spreading strip (PSS) materials are preferably compliant, soft feeling, and non-irritating to the wearer's skin and may be manufactured from a wide range of materials, such as porous foams; reticulated foams; apertured plastic films; or woven or nonwoven webs of natural fibres (e.g. wood or cotton fibres), synthetic fibres (e.g. polyester or polypropylene fibres), or a combination of natural and synthetic fibres. If they include fibres, the fibres may be spunbonded, carded, wet-laid, meltblown, hydroentangled, or otherwise processed as is known in the art. They may be a composite material, such as when comprising an open net or scrim structure in combination with a spunbonded web.

Preferably, the SPS exhibits a low tendency for the passage of faeces. Optionally, the SPS may exhibit a z- or thickness directional gradient structure, or be a laminate or composite material, such as exhibiting particularly skin friendly properties on the user oriented surface, or particular faeces absorbent properties on the opposite surface, which may be particularly beneficial in the context of low viscosity or "runny" faeces.

Optionally, the SPS may be a composite material, such as being made in stripes (y-directional variation) or connected patches (x-directional variation). Optionally, the SPS may comprise additives, such as skin friendliness enhancer, such as emollients or the like, as well known in the art.

For certain executions, it may be preferred that the SPS is fluid impermeable, e.g. as a hydrophobic nonwoven, a film, or a composite thereof.

For specific executions of the longitudinal extensions of the various regions of the SPS and PSS, express reference is made to the above referenced WO' 109, except for making of the absorbent cores.

The one or more opening(s) through the core may be applied concurrently with applying the openings in the SPS and PSS, as may preferably by applying a separation line, such as a slit, thereto. Preferably, the absorbent core is free of particulate material, such as without limitation superabsorbent particles, in the regions where the opening is created so as to minimize or even completely avoid losses of the particulate material. Further, an absorbent core according to the present invention preferably exhibits a liquid handling structure and a liquid storage structure, which may be positioned in different relative arrangement, as explained in the above, as may be achieved by conventional means during the manufacturing,

### Test methods

All testing should be performed in a conditioned room maintained at about 23 +/- 2 °C and about 50 +/- 2 % relative humidity.

### Free Swell Absorption Capacity Test

Following the Test Method PA07/05 of Hygiene-Technologie GmbH (Hy-Tec), Haan, Germany, the test specimen is submersed in sufficient 0.9 w-% saline for 30 mins, after which it is removed and placed on a coarse grid or sieve, e.g. a grid with 1 cm wide openings and a ribs of about 2 mm. After a further waiting time of 10 mins, the weight of the test specimen is recorded as free swell absorbency in grams.

### Centrifuge Capacity Test

This test is generally following the Edana NWSP 241.0.R2 (15) Polyacrylate Superabsorbent Powders - Determination of the Fluid Retention Capacity in Saline Solution by Gravimetric Measurement Following Centrifugation, but applying it to an absorbent article, as per Test Method PA07/05 of Hygiene-Technologie GmbH (Hy-Tec), Haan, Germany. To this end, a test sample as treated according to the free swell absorption test is positioned within a commercial centrifuge exerting a centrifugal force of about 250 times gravity (e.g. by having an internal diameter of 225 mm at a rotation of 1400 rpm) for 3 mins. The test specimen is removed and weighed and the centrifuge absorbent capacity recorded in grams.

## Claims

1. A hygiene article for being worn on the lower torso of a wearer
- said article exhibiting
∘ a front and a rear waist region
■ along a length / longitudinal / x - direction
■ and separated by a crotch point region comprising a crotch point adapted to be positioned in the perineal region of the wearer during use;
∘ a width / cross-directional / y-direction, perpendicular thereto and corresponding to the left-right direction of the wearer;
∘ a z- or thickness direction perpendicular to the x- and y- direction,
- said article comprising
∘ a skin protection sheet (SPS) adapted to separate bodily exudates from the skin of a wearer,
■ said SPS comprising
• a rear opening adapted to be positioned in registry with the anus and
• a front opening to be positioned in registry with a bodily exudate releasing body opening or a genital organ,
• whereby said rear and front opening do not extend into each other and are positioned opposite to each other relative to the crotch point of the article,
∘ a backsheet adapted to retain said bodily exudates,
∘ an absorbent core adapted to absorb liquids of said bodily exudates and being positioned z-directionally between said SPS and said backsheet,
- further comprising a pull and spreading strip (PSS) adapted to
∘ urge said SPS into the anal cleft of a wearer during the article's intended use,
∘ and to maintain simultaneously said first and said second opening(s) of said SPS cross-directionally open during the article's intended use,
- **characterized in that** said absorbent core
∘ extends from said crotch point region longitudinally towards and under, optionally beyond at least one, preferably both of said front and rear opening of said SPS,
∘ comprises at least one core opening in registry with at least one of said front and rear opening(s) of said SPS,
∘ and is connected to said SPS and directly or indirectly to said PSS in the proximity of said opening(s)
■ preferably by melt-fusion bonding, more preferably ultrasonic bonding,
∘ such that said opening of the absorbent core is maintained cross-directionally open upon the intended use, so as to allow exudates to pass through said SPS and said absorbent core.

2. A hygiene article according to claim 1, wherein said article exhibits a first manufacturing configuration wherein essentially all elements are essentially planar, and a second in-use configuration adopting a body conforming U- shape, by said longitudinally extending center line extending from the front or rear waist region of a wearer through the crotch point region into the opposite waist region of the wearer.

3. A hygiene article according to any of claims 1 to 2, wherein said openings are formed by separation lines extending predominantly longitudinally, preferably parallel to and in the proximity of a longitudinal centerline of said article in the manufacturing configuration.

4. A hygiene article according to any of claims 1 to 2, wherein said openings are formed by removing materials from said absorbent core, SPS, and PSS.

5. A hygiene article according to any of the preceding claims, wherein said absorbent core is essentially free of particulate material in the cross-directional proximity of said openings.

6. A hygiene article according to any of the preceding claims, wherein said absorbent core comprises a seal line in the proximity of said separation lines.

7. A hygiene article according to any of the preceding claims, wherein said absorbent core comprises a first surface adapted for handling urine and an opposite surface adapted for handling faeces.

8. A hygiene article according to any of the preceding claims exhibiting a capacity of more than about 50 ml, or more than about 100 ml, or more than about 200 ml, or more than about 300 ml or more than about 400 ml, or more than about 500 ml, or less than about 2000 ml, determined according to the Centrifuge Capacity Test.

9. A hygiene article according to any of the preceding claims, wherein said SPS is selected from the group consisting of hydrophilized nonwoven material, hydrophobic nonwovens, aperture nonwovens, films, apertured films, and combinations thereof.

10. A hygiene article according to any of claims 1 to 9, wherein
- said core opening is in registry with said front SPS opening;
- said core is attached to said SPS in the proximity of said front SPS opening;
- said core is essentially unattached to said SPS in the proximity of said rear SPS opening.

11. A hygiene article according to any of claims 1 to 9, wherein
- said core opening is in registry with said rear SPS opening;
- said core is attached to said SPS in the proximity of said rear SPS opening;
- said core is essentially unattached to said SPS in the proximity of said front SPS opening.

12. A hygiene article according to any of claims 1 to 9, wherein
- said core comprises a front and a rear opening in registry with said respective SPS openings;
- said core is attached to said SPS in the proximity of said front and rear openings;
- said article further comprises an exudate separation sheet (ESS)
- connected to said core in the crotch region longitudinally between said front and rear openings
- positioned between said absorbent core and said backsheet,
- extending longitudinally forward or rearward so as to extend over one of said front and rear openings, but not the other.

13. A hygiene article according to any of claim 12 comprising an exudate separation sheet exhibiting an absorbent capacity of more than about 100 ml/m².
